# EUROPEAN PATENT APPLICATION

(11) **EP 3 909 501 A1**
(43) Date of publication of application: **17.11.2021**
(21) Application number: 20173860.6
(22) Date of filing: 11.05.2020
(51) Int. Cl.: A61B 5/022, A61B 5/00

(54) **APPARATUS AND METHOD FOR IMPROVED BLOOD PRESSURE MEASUREMENT**

(71) Applicant: Koninklijke Philips N.V., 5656 AG Eindhoven (NL)
(72) Inventor: SCHLACK, Andreas Wolfgang, 5656 AE Eindhoven (NL)
(74) Representative: Philips Intellectual Property & Standards

(57) **Abstract**

An apparatus (10) is for use with an inflatable cuff (14) for performing oscillometric blood pressure measurements, and the apparatus configured for detecting slippage of a cuff fastening (15). The apparatus includes a control unit (12) in combination with an acoustic sensor (20). The acoustic sensor is arranged so as to be sensitive in use to sounds emanating from the cuff during inflation of the cuff for performing a blood pressure measurement. A control unit acquires from the cuff a pressure signal indicative of pressure in the cuff, the pressure signal for oscillometrically deriving a blood pressure measurement. The control unit is further configured to receive a sensor input from the acoustic sensor and process it to detect sound signatures in the signal corresponding to a slippage of a fastening arrangement of the cuff, to thereby detect occurrence of cuff fastening slippage events. The control unit is then configured to trigger a response action responsive to detecting any slippage event, which could be processing the pressure signal to detect and compensate any artifacts in the signal caused by the slippage event, or could be for labelling the acquired measurement to alert a user that it could be compromised.

## Description

### FIELD OF THE INVENTION

The present invention relates to an apparatus and method for use with a blood pressure measurement cuff for measuring blood pressure, for improving accuracy of blood pressure measurements.

### BACKGROUND OF THE INVENTION

Blood pressure (BP) or, more precisely, arterial blood pressure, is the pressure exerted by circulating blood on the arterial vessel walls. Blood pressure is a periodic signal, which rises at each contraction of the heart and decreases between heart beats. It is typically described by systolic blood pressure (SBP), diastolic blood pressure (DBP) and mean arterial blood pressure (MAP), where systolic blood pressure is the maximum blood pressure during a heart cycle, diastolic blood pressure is the minimum blood pressure during the heart cycle, and mean arterial blood pressure is the average blood pressure during a heart cycle.

Different techniques exist by which blood pressure can be determined and these can be classified as invasive or non-invasive measurement techniques. Typically, non-invasive blood pressure (NIBP) measurement techniques are cuff-based. In cuff-based measurement, an inflatable cuff is placed around a limb (usually the upper arm) of a subject. The pressure in the cuff is then varied through a range of applied pressures, and blood pressure inferred. There are two main methods by which blood pressure can be derived using the cuff-based approach: the auscultatory method and the oscillometric method.

The auscultatory method for blood pressure measurement is based on the appearance and disappearance of sounds created by the artery under the cuff during the period that the cuff pressure is changed. These sounds are referred to in the art as Korotkoff sounds. The pressures at which the Korotkoff sounds appear and vanish are indicative of DBP and SBP with Korotkoff sounds appearing at each heart beat between DBP and SBP. The measurement of sound can be performed manually with a stethoscope that is placed over the artery just below the cuff, or in an automated way with a microphone under the cuff.

In the oscillometric method, systolic and diastolic blood pressure values can be derived from small volume oscillations or pressure oscillations that are induced in the cuff by each heartbeat. The amplitude of these volume or pressure oscillations depends upon the difference between the cuff pressure and the actual arterial blood pressure (the transmural pressure). Oscillations in transmural pressure across the artery wall can be measured. The amplitudes of these oscillatory signals can be used to derive an indication of systolic and diastolic blood pressure. Systolic blood pressure and diastolic blood pressure can be determined for example as the cuff pressure values at points where the volume or pressure oscillations have amplitudes of a certain fraction of the maximum oscillation amplitude. These fractions are typically heuristically determined.

In both the auscultatory and oscillometric method, the mean arterial pressure is typically calculated as: MAP = (2/3^{∗}DBP) + (1/3^{∗}SBP).

The oscillometric and auscultatory measurement methods can be performed either during inflation of the cuff (i.e. with gradually increasing applied pressures) or during deflation of the cuff (gradually decreasing applied pressures).

Conventionally, measurements during deflation are used, in which the cuff is rapidly inflated to a level above the SBP, so that the blood flow in the artery under the cuff is fully occluded, after which cuff pressure is decreased gradually, or in a stepwise manner. During deflation, the volume or pressure oscillations, or the Korotkoff sounds, are measured.

While deflation stage measurement is well-established, it can produce discomfort. The subject is exposed to a relatively high cuff pressure for a period of time, and pressures above a certain level can be uncomfortable and even painful, either due to the pressure exerted by the cuff itself or due to a build-up of venous blood in the clamped extremity (namely, venous pooling). The longer these pressures are applied to the subject, the higher the discomfort level.

A further disadvantage of deflation-based measurement is that the process of inflating the cuff and then deflating the cuff can be fairly long in duration, each measurement during deflation typically taking 40 seconds to complete. Also, since a defined maximum pressure level needs to be achieved before the deflation procedure can be initiated, the subject is exposed to a maximum cuff pressure that is higher than that required for the blood pressure measurement itself. Furthermore, the inherent variability of blood pressure over time can distort a single blood pressure measurement.

Due to these issues, alternative systems are known which determine oscillations during inflation of the blood pressure cuff. These devices can reduce the discomfort as blood pressure measurement may be accomplished in less time using the inflation stage compared to the deflation stage. To minimize the measurement time without sacrificing accuracy, the cuff inflation speed can be made pulse-rate dependent such that measurement encompasses the optimal number of oscillations (i.e. enough to ensure a certain accuracy but no more). Thus, once the pressure range of interest is achieved at the inflation stage, pressure relief may be immediately initiated.

However, in general, non-invasive blood pressure (NIBP) measurements are vulnerable to distortions in the measurements. This is due to the small amplitude of the pressure oscillations which are being measured which means that small perturbations can lead to noise artifacts which saturate the signal being measured.

One such distortion relates to the sudden recuperation of volume that often occurs during inflation of the blood pressure cuff. The recuperation of volume is an effect that is related to the blood pressure cuff (gradually) becoming pressurized during inflation. During this pressurization, the material that holds the cuff around the arm needs to generate a higher counter force per area. Although this prevents the cuff from falling off during a measurement, the cuff may not be secured tightly enough at local parts and thus may come loose. For example, the fastening (e.g. Velcro) that holds the cuff in place may detach in local areas during inflation. When this happens, the volume of the cuff expands locally. This is known as slippage of the cuff fastening. This produces a characteristic sound (known as a "cracking" sound).

These sudden mechanical movements of the cuff fastener (e.g. Velcro fastening), known as cuff cracks, result in oscillations in the cuff pressure signal which can easily be confused with pulsatile oscillations. If such cuff-cracks occur during inflation of the cuff (during the oscillometric measurement), the cuff pressure can falsely be interpreted as mean arterial pressure, which would lead to a false low NIBP-measurement.

Also, during pressurization of the cuff, the inflatable bladder of the cuff (which is usually an elastic material such as a plastic or rubber) expands. At the initiation of a blood pressure measurement, the bladder of the cuff can comprise small folds that result in parts of the bladder volume being occluded. During inflation, the bladder expands and this can result in the unfurling of the folds, which then also produces a recuperation of volume and a sudden expansion in the cuff.

These effects thus can result in cuff slippage, which introduces an artifact in the pressure signal acquired from the wearable cuff.

As such, existing inflation based techniques that measure blood pressure from this pressure signal, can lead to inaccurate (e.g. false) blood pressure measurements. Inaccurate measurements can lead to an incorrect diagnosis and/or clinically inappropriate responsive action.

US 8,911,378 discloses a method for determining that a cuff slippage condition exists. However, this approach relies on a fairly complex signal processing method in which a level of noise in the signal is determined using various algorithms. This method is also inherently unreliable, since noise can be caused by various factors, not only cuff slippage, and thus cuff slippage can be falsely detected.

An improved approach to addressing the above discussed problems associated with cuff slippage would therefore be of advantage.

### SUMMARY OF THE INVENTION

The invention is defined by the claims.

According to examples in accordance with an aspect of the invention, there is provided an apparatus for use with a wearable cuff in determining blood pressure and/or pulse rate, wherein the wearable cuff is inflatable for pressurizing a measurement site of the subject, the apparatus comprising:
an acoustic sensor for detecting sounds originating from the blood pressure measurement cuff (in particular sounds corresponding to slippage of a fastening arrangement of the cuff); and
a control unit operatively coupleable with the cuff and configured to:
   acquire a pressure signal indicative of a pressure in the wearable cuff during inflation of the cuff,
   receive sound signals from the acoustic sensor,
   analyze the sound signals to detect sounds corresponding to slippage of a fastening arrangement of the cuff; and thereby identify occurrence of cuff fastening slippage events, and
   trigger a response action responsive to detecting any cuff fastening slippage events,
   wherein the control unit is further operable to determine the blood pressure of the subject using an oscillometric technique, by analyzing oscillations in the acquired pressure signal.

Thus embodiments of the invention are based on use of a sound sensor (e.g. microphone) to detect occurrence of slippage of the cuff fastening. Depending upon the type of fastening, the slippage event will produce a characteristic sound, which sound can be reliably detected in the acquired sound sensor signals. Thus, for example, the timing and duration (and even intensity) of cuff slippage events can be detected (preferably in real time) and this can be used to trigger a response action aimed at mitigating any distortions in the acquired pressure signal which result from this. For example, the pressure signal could be processed to correct or compensate any artifacts which have appeared in the signal as a result of the slippage event. Alternatively, the acquired measurement could be labelled to alert a user who later retrieves the measurement that the measurement could be inaccurate. Other response actions are also possible, as will be discussed later.

By using a microphone, a separate modality is used to detect the slippage events from the acquired cuff pressure signal itself. This means that slippage event detection is more reliable, since there is no possibility that the detected acoustic signals could be caused by other sources of pressure-signal noise which can occur in the pressure signal itself. The detection of the slippage event is thus independent of other distortions in the pressure signal. This method is also relatively simple computationally, as it relies only on detecting certain characteristic patterns or signatures in the sound signal indicative of the slippage event; it does not rely on complex signal analysis techniques applied to the pressure signal.

Depending upon the nature of the triggered response action, the blood pressure measurement could be determined before performing the response action or afterward. For example if the response action comprises adjusting or compensating the acquired pressure signal for any slippage event artifacts, then this response action would be done in advance of determining the blood pressure measurement. Alternatively, if the response action comprises simply labelling the derived blood pressure measurement in the event of a slippage event, this would be done after deriving the blood pressure measurement.

As mentioned above, often, blood pressure cuffs use hook and loop (Velcro) type fastenings to secure them (although other fastening types also exist).

Thus, according to at least one set of embodiments, the apparatus may be for use with a wearable cuff which comprises a hook and loop type fastening arrangement (such as Velcro or similar), and wherein the control unit is configured to detect in the sound signals sounds corresponding to slippage of a hook-and-loop type fastening.

As mentioned above, the slipping of hook and look type fastenings generates a highly characteristics sound, which is referred to in the art as a 'cracking' sound. The sound of hook and loop fastening slippage is very specific because of the chain reaction when some hooks and loops of a Velcro fastener detach and re-attach. This sound is thus reliably recognizable in a sound recording acquired from the vicinity of the cuff.

In a preferred set of embodiments, the acoustic sensor may be integrated in the control unit, i.e. located in or comprised by the control unit.

The control unit is a distinct structure from the cuff, designed to be physically separated from the cuff for example. The two in use are linked by a control line and/or a fluid supply line. For example the control unit may comprise a housing containing one or more processor or controllers, and wherein the microphone is integrated in the housing of the control unit.

The acoustic sensor may be acoustically coupled with the cuff via a physical connection established in use between the control unit and the cuff, e.g. a control line and/or fluid supply line. The sound sensor can be arranged to pick up sounds transmitted along such a connection in order to listen for the characteristic cuff slippage sounds.

By integrating the acoustic sensor in the control unit and not in the cuff, this avoids the microphone picking up other sound signals generated by phenomena and movements associated with the cuff which are not associated with slippage. For example, it avoids the sound sensor picking up the Korotkoff sounds from the artery, or picking up sounds associated with normal inflation of the cuff. The cuff slippage is a fairly loud or acoustically intense event, and thus can be detected via the indirect acoustic coupling provided by a connection line between the control unit and the cuff. By locating the acoustic sensor in the control unit, this also means it is close to the processing components of the control unit, thus avoiding the need to run a long signal connection between for example a sound sensor integrated in the cuff and the control unit.

By way of one set of examples, the acoustic sensor may be arranged in the control unit in acoustic communication with a connection port, the connection port for connecting the control unit to the cuff in use. The connection port may be for receiving an end connector of a connection line that connects the control unit to the cuff.

The control unit with acoustic sensor can be provided by itself, and designed to make connection with an already existing cuff. It thus typically includes a port or socket via which it can connect to a cuff to control it and/or inflate it. Thus when in use, the acoustic sensor may be arranged to be in acoustic communication with a connection line arranged to connect between the control unit and the cuff.

The acoustic communication can be facilitated by arranging the acoustic sensor in direct or indirect physical communication/contact with the connection port for example. Thus the two are arranged directly or indirectly physically coupled (in solid physical communication). A solid acoustic communication path between the acoustic senor and the connection port is thus provided.

In some examples, the acoustic sensor may be arranged in the control unit in acoustic communication with a fluid supply connection for fluidly connecting to the wearable cuff in use for supplying fluid for controlling inflation of the cuff. Preferably the fluid is air to avoid mess in case of leakage of the fluid, however, liquid can also be used as the fluid. The fluid supply connection could be a fluid (e.g. air) supply outlet port for example, for receiving an end connector of a fluid supply line between the control unit and the cuff. Thus, in use, the acoustic sensor would be arranged in acoustic communication with such a fluid supply line to the cuff, e.g. tube or pipe.

This fluid supply line may typically have a certain minimal physical thickness or size to accommodate air under pressure. This therefore makes it a good element to use for coupling sounds from the cuff to the control unit, since an effective solid coupling between the sound sensor and the cuff is thereby established.

According to one or more embodiments, the analyzing of the sound signals to detect the slippage events may comprise detecting in the acquired sound signals one or more pre-determined signal features or characteristics associated with cuff slippage. It may comprise detecting one or more pre-determined cuff-slippage signatures or fingerprints in the signals.

For example, the control unit comprises a memory, and the memory has pre-stored one or more signal features, characteristics or other signal signature or fingerprint which is known to be detectable in the acquired sound signals in the event of a slippage event. These can then be retrieved and used when analyzing patterns in the signals to determine whether a slippage sound event is present in the acquired sound signal.

According to one or more embodiments, the analyzing the sounds signals to detect the slippage events may comprise a frequency-based analysis and detection, i.e. detecting the sound signal components in the sound signals corresponding to the slippage events based on their frequency.

According to one or more embodiments, the analyzing the sound signals to detect the slippage events may comprise one or more steps of processing the sounds signals.

According to one or more embodiments, the analyzing the sound signals to detect the slippage events may comprise applying a filter to the sounds signals, having a frequency set (in advance) based on knowledge of a typical frequency range of the slippage event for the particular fastening arrangement in question. In examples for instance in which the fastening arrangement is a hook and loop type fastening, the filter may be a high-pass filter. It is known that slippage events associated with slipping of a hook and loop fastening is characterized by high frequencies.

According to one or more embodiments, the analyzing the sound signals to detect the slippage events may be based on use of a machine learning algorithm, such as a convolutional neural network (CNN). The algorithm may be an algorithm that has been trained using training data comprising previously acquired acoustic signals, each labelled according to whether or not it comprises a signature of a slippage event.

In accordance with one or more embodiments, the response action may comprise processing the acquired pressure signal to identify one or more artifacts in the pressure signal corresponding to the detected cuff fastening slippage events. This may comprise processing oscillations in the signal for example.

Identifying the artifacts in the pressure signal may be based on correlating timings of the detected slippage events to the acquired pressure signal to detect signal elements (e.g. signal oscillations) which temporally correspond with the slippage events.

The response action may in some examples comprise processing the acquired pressure signal to compensate for the detected artifacts.

For example, in some embodiments, the compensating may comprise:
discarding oscillations in the acquired pressure signal at one or more occasions where the detected artifact occurs; and
applying a correction to the acquired pressure signal at one or more occasions where the detected artifact occur.

The apparatus may in some examples be configured to apply the correction to the acquired pressure signal by: subtracting the detected artifact from the raw data of the acquired pressure signal at one or more occasions where the detected artifact occurs to acquire a corrected pressure signal.

Additionally or alternatively, according to one or more embodiments, the response action may comprise adjusting a duration or repetition count of the blood pressure measurement performed using the cuff.

Additionally or alternatively, according to one or more embodiments, the response action may comprise recording occurrences of slippage events and labelling acquired pressure measurements which overlap with detected slippage events. This therefore can alert a person reviewing the measurements that the measurement may be inaccurate.

In accordance with one or more embodiments, the control unit may comprise a fluid (e.g. air) supply means, and a fluid (e.g. air) supply outlet port for fluidly connecting to the wearable cuff in use. The control unit may be configured to implement an oscillometric blood pressure measurement using the wearable cuff, based on controlling the inflation of the cuff, and acquiring the pressure signal indicative of the pressure inside the cuff.

Examples in accordance with a further aspect of the invention provide a system which comprises:
a wearable blood pressure measurement cuff, and
an apparatus in accordance with any example or embodiment outlined above or described below, or in accordance with any claim of this application, the control unit of the apparatus arranged operatively coupled with the blood pressure measurement cuff for acquiring from the cuff the pressure signal.

Examples in accordance with a further aspect of the invention provide a method for use in determining a blood pressure and/or pulse rate measurement using a wearable cuff, wherein the wearable cuff is inflatable for pressurizing a measurement site of the subject, the method comprising:
acquiring sound signals corresponding to sounds originating from the blood pressure measurement cuff;
acquiring from the wearable cuff a pressure signal indicative of a pressure in the wearable cuff during inflation of the cuff,
analyzing the acquired sound signals to detect sounds corresponding to slippage of a fastening arrangement of the cuff, and thereby identify occurrence of cuff fastening slippage events;
triggering a response action responsive to detecting any cuff fastening slippage events; and
determining a blood pressure measurement of the subject using an oscillometric technique, by analyzing oscillations in the acquired pressure signal.

Examples in accordance with a further aspect of the invention provide a computer program product comprising code means configured, when run on a processor, to cause the processor to perform a method in accordance with any example or embodiment outlined above or described below, or in accordance with any claim of this application,
wherein said processor is a processor operatively coupled with a wearable blood pressure measurement cuff, and further operatively coupled with an acoustic sensor for acquiring sounds originating from the wearable cuff.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 schematically illustrates an example apparatus in use with a blood pressure measurement cuff;
Fig. 2 schematically illustrates components of an example apparatus according to one or more embodiments; and
Fig. 3 outlines in block diagram form an example method according to one or more embodiments.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides an apparatus for use with an inflatable cuff for determining oscillometric blood pressure measurements, the apparatus configured for detecting slippage of a cuff fastening. The apparatus includes a control unit in combination with an acoustic sensor. The acoustic sensor is arranged so as to be sensitive in use to sounds emanating from the cuff during inflation of the cuff for performing a blood pressure measurement. A control unit acquires from the cuff a pressure signal indicative of pressure in the cuff, the pressure signal for oscillometrically deriving a blood pressure measurement. The control unit is further configured to receive a sensor input from the acoustic sensor and process it to detect sound signatures in the signal corresponding to a slippage of a fastening arrangement of the cuff, to thereby detect occurrence of cuff fastening slippage events. The control unit is then configured to trigger a response action responsive to detecting any slippage event, which could be processing the pressure signal to detect and compensate any artifacts in the signal caused by the slippage event, or could be for labelling the acquired measurement to alert a user that it could be compromised.

To enable better understanding of embodiments of the invention, the procedure for performing a standard blood pressure measurement will first be described.

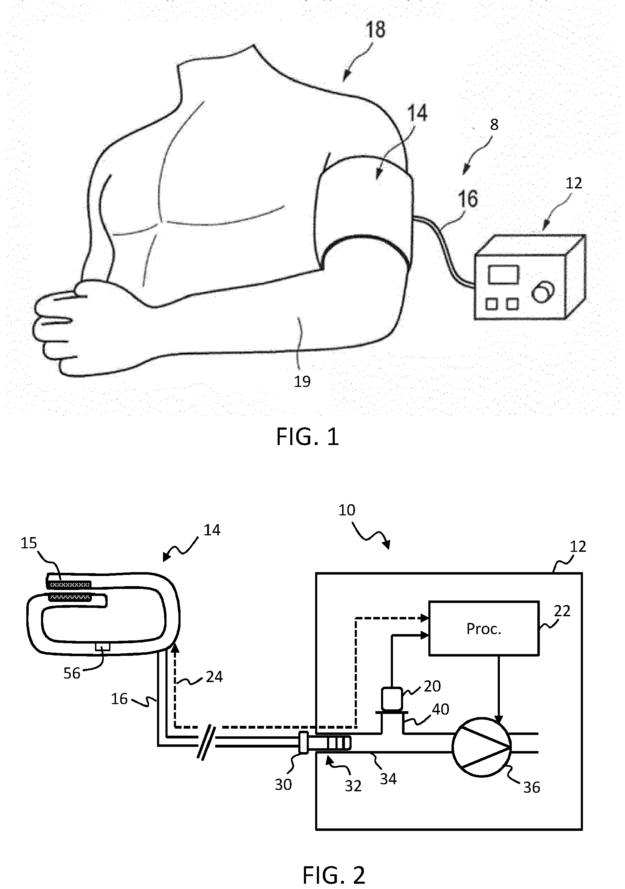

Fig. 1 schematically illustrates the components of an example cuff-based blood pressure measurement system 8. The system comprises a fluid-inflatable cuff 14 which is attachable around a limb of a subject 18, most typically the arm 19. The system further comprises a control unit 12 which couples to the cuff via a connection line 16. The control unit typically both controls inflation of the cuff, and monitors pressure signals from a pressure sensor integrated in the cuff bladder to determine the blood pressure measurements. Thus, the control unit may typically comprise a fluid supply means, e.g. a fluid pump (not shown in Fig. 1), and wherein the connection line 16 is a fluid supply line along which fluid is pumped by the control unit to control a level of inflation of the cuff to perform a blood pressure measurement. The fluid is typically air, but use of liquid is also possible. The connection line also integrates a data communication line for example.

To perform an oscillometric blood pressure measurement, the cuff 14 is first wrapped around the arm 19 of the subject, and fastened to the arm using the fastening means (e.g. hook and loop fastening means such as Velcro).

The control unit then varies an inflation level of a fluid-inflatable chamber of the cuff 14 across a range of pressures to thereby vary a pressure applied to the part of the body to which the device is mounted. In particular, the fluid-inflatable chamber of the cuff 14 may be gradually inflated so as to increase a pressure applied to the artery through a range of increasing pressures.

At each of one or more of the applied pressures, an internal chamber pressure is monitored and a pressure signal acquired indicative of pressure oscillations inside the inflatable chamber. For a given (fixed) chamber inflation, such oscillations can be taken to be representative of variations in pressure applied to the device by the underlying artery as it pulses with blood. These oscillations may hence be taken as representative of oscillations in arterial pressure of the underlying artery.

By measuring these oscillations at different applied pressures, the arterial pressure at different transmural pressures is obtained, allowing a full blood pressure measurement to be performed.

A pressure sensor may be included in the cuff 14 fluid chamber for monitoring a pressure inside the internal chamber.

An example apparatus in accordance with one or more embodiments is schematically illustrated in cross-section in Fig. 2.

The apparatus 10 is for use with a wearable cuff 14 in determining blood pressure and/or pulse rate. As in the explanation above, the wearable cuff 14 is inflatable for pressurizing a measurement site of the subject, such as an arm.

The apparatus 10 comprises a control unit 12 within which is integrated a processor unit 22. The control unit is arranged to be connected in use with the wearable fluid-inflatable cuff for deriving blood pressure measurements.

The cuff 14 comprises a fastening arrangement 15 by which the cuff is releasably fastened to a limb of a subject during use. In preferred embodiments, the fastening arrangement comprises a hook and loop type fastening arrangement (such as Velcro).

In particular, the control unit is arranged for communicatively coupling with the cuff 14 via a data communication line 24. Using this line, the processor 22 of the control unit is configured to acquire a pressure signal indicative of a pressure in the wearable cuff during inflation of the cuff.

In the present example, the control unit is also configured for controlling the inflation level of the cuff 14 for performing blood pressure measurements. In particular, the control unit is arranged for making fluid connection with the cuff in use for controlling a fluid inflation level of the cuff 14. The fluid is typically air but liquid can alternatively be used. This allows the control unit to implement the oscillometric blood pressure measurement by controlling the cuff to vary through a range of different pressures. A fluid supply line 16 extends from the cuff and terminates at a fluid connector 30. The connection port 32 of the control unit 12 is arranged to receive the connector 30 to fluidly connect an internal fluid supply line 34 of the control unit to the external fluid supply line 16 of the cuff. In this example, the control unit 12 further comprises a fluid pump 36 configured to supply pressurized fluid along internal fluid supply pipe 34 to the fluid outlet port 32. The pressured fluid is then coupled along external fluid supply pipe 16 to the cuff 14 for pressurizing the fluid chamber of the cuff.

The data communications line 24 may connect to the control unit 12 via the same connector 30 by which it is fluidly connected to the control unit. For example the fluid supply line 16 may have an integrated data communication line 24 so that data and fluid can be transported along the same single line.

The apparatus 10 further comprises an acoustic sensor 20 arranged for detecting sounds generated by the blood pressure measurement cuff. In particular it is for the purpose of detecting sounds corresponding to slippage of the fastening arrangement 15.

The acoustic sensor 20 is preferably adapted for detecting sub ultrasonic sound signals only. It may comprise one or more acoustic transducers. It may comprise one or more microphones for example.

In this example, the acoustic sensor 20 is integrated inside the control unit. In particular, the acoustic sensor is arranged acoustically coupled with the fluid connection port 32 of the control unit via the internal fluid supply pipe 34. In particular, a stunted sub-branch 40 of the internal fluid supply pipe is provided extending a short distance transverse the general longitudinal direction of the internal supply pipe 34, and capped across its end. The acoustic sensor 20 is arranged acoustically coupled with this sub branch 40. In this way the acoustic sensor is arranged in solid physical communication with the supply pipe 34 which leads to the fluid outlet port 32. Thus, the acoustic sensor is indirectly physically or solidly coupled with the fluid connection port 32.

When the fluid supply line 16 of the cuff 14 is connected into the outlet port 32 of the control unit 12, this means that the acoustic sensor 20 is arranged acoustically coupled with the cuff 14 via the solid physical intermediary of the internal supply pipe 34 in combination with the external fluid supply pipe 16. Thus, in use, sounds travel from the cuff, along the cuff inflation tube 16 and are received by the sound sensor 20.

Although in the example of Fig. 2, the acoustic sensor is provided within the control unit 12, this is not essential. In other examples the acoustic sensor 20 may be provided outside of the control unit. In some examples, it may be provided on an outside of the housing of the control unit, for listening to sounds emanating from the cuff 14 across the air gap between them. In further examples, an acoustic sensor 20 may be provided as a separate external unit, for example for positioning adjacent the control unit 12, or for connecting to an object within the environment such as a table or bed, or even for connecting to the cuff itself.

However, in preferred examples, the acoustic sensor is configured to be arranged physically and spatially separated from the cuff 14 during use so as to avoid the sound sensor picking up a large number of sounds which are not related to slippage of the fastening arrangement. For example, in the case that the acoustic sensor 20 is attached to the cuff 14, the acoustic sensor is likely to detect sounds related to the normal inflation of the cuff, which sounds are not relevant for the purposes of detecting cuff slippage. If the sensor is instead spatially apart from the cuff 14, fewer of the sounds will be detected. If the sensor is integrated in the control unit 12, even fewer of these non-relevant sound signals will be picked up, which makes detecting the slippage more reliable.

The control unit may comprise a housing or casing within which the processor 22, the pump 36, and the acoustic sensor 20 are located.

As mentioned, the processing unit 22 of the control unit 12 is configured to communicate with the cuff 14 during inflation of the cuff via the communication line 24 to acquire a pressure signal corresponding to a pressure of the cuff during the inflation.

The processing unit 22 of the control unit 12 is further configured to receive sound signals from the acoustic sensor 20 during said inflation of the cuff 14.

The processing unit 22 of the control unit 12 is configured to analyze the sound signals to detect sounds corresponding to slippage of a fastening arrangement of the cuff, and thereby identify occurrence of cuff fastening slippage events.

The processing unit 22 of the control unit is also configured to trigger a response action responsive to detecting any cuff fastening slippage events.

Further to the above, the processing unit 22 of the control unit is further operable to determine the blood pressure of the subject using an oscillometric technique, by analyzing oscillations in the acquired pressure signal. Depending upon the nature of the response action, the step of deriving a blood pressure measurement may be performed before the response action or afterwards. For example, if the response action is to detect one or more artefacts in the acquired pressure signal, the blood pressure measurement is derived after performing this action. Alternatively, the response action is simply the labelling of acquired blood pressure measurements to indicate that they overlap with a slippage event, the blood pressure measurement may be acquired before performing this response action.

It is further noted that although in the example of Fig. 2, the control unit is configured to control the inflation of the blood pressure measurement cuff 14, this is not essential. In other embodiments, a separate unit might be provided for controlling the fluid inflation of the cuff. Thus, inclusion of a fluid pump 36, and fluid outlet pipe 34, 32 in the control unit is not essential.

The detecting the occurrence of slippage events in the sounds signals can be done in different ways.

In accordance with at least one set of embodiments, sound signals are analyzed and the slippage events detected based on detecting in the acquired sound signals one or more pre-determined signal features or characteristics associated with cuff slippage, or one or more pre-determined cuff-slippage signatures or fingerprints in the signals.

For example, the control unit may comprise a memory, and wherein the memory has pre-stored one or more signal features, characteristics or other signal signature or fingerprint which is known to be detectable in the acquired sound signals in the event of a slippage event. These reference signal features or fingerprints may be determined in advance, for example empirically, based on recording various instances of slippage of the specific fastening means 15 comprised by the cuff 14, and extracting from these recordings common characteristic signal features or patterns which can be used in future to recognize occurrence of the fastening slippage in newly recorded sound signals.

An algorithm is applied configured to process the sound signal to detect any of these signal features, or characteristics or fingerprints/signatures in the acquired sound signal.

Thus the algorithm used to detect the slippage events can be tailored for detecting the sounds associated with the fastening mechanism of the specific cuff to be used with the apparatus. Thus, the apparatus may be designed for use with a specific type of cuff. In some examples, the control unit 12 may be configured with multiple modes, wherein each mode is for detecting sounds associated with different type of cuff attachment means. A user may switch between the modes in accordance with the type of cuff they are using with the apparatus. In this way, the apparatus can be operable with a range of different blood pressure measurement cuffs.

According to one or more embodiments, the analyzing the sounds signals to detect the slippage events may comprise a frequency-based analysis and detection, i.e. detecting the sound signal components in the sound signals corresponding to the slippage events based on their frequency.

According to one or more embodiments, the analyzing the sound signals to detect the slippage events may comprise one or more steps of processing the sounds signals.

According to one or more embodiments, the analyzing the sound signals to detect the slippage events may comprise applying a filter to the sounds signals, having a frequency set (in advance) based on knowledge of a typical frequency range of the slippage event for the particular fastening arrangement in question. In examples for instance in which the fastening arrangement is a hook and loop type fastening, the filter may be a high-pass filter. It is known that slippage events associated with slipping of a hook and loop fastening is characterized by high frequencies.

A filter can extract from the sound signals any signal components in a frequency range that matches the typical frequency of slippage events for the particular fastening arrangement in question. In some examples, a further threshold may be applied as a condition for slippage event detection. For example, if the extracted signal components exceed a certain amplitude threshold, or duration threshold, detection of a slippage event may be recorded.

According to any method of detection, a timing of each of the detected slippage events may be recorded. A duration of each of the detected slippage events may be recorded. An intensity (e.g. relative intensity) of each of the slippage events may be recorded. These parameters may in some examples be used as part of the response action to follow.

In a further set of embodiments, the analyzing the sound signals to detect the slippage events is based on use of one or more machine learning algorithms. In this case for example, the algorithm may be trained in advance using training data comprising a set of previously acquired acoustic signals (e.g. sound recordings), each labelled according to whether or not the sounds recorded in it contain a slippage event. It might further be labelled according to one or more of: a duration of the slippage event captured by it, a timing of the duration event, and a relative intensity of the slippage event.

A machine-learning algorithm is any self-training algorithm that processes input data in order to produce or predict output data. Here, the input data comprises sound signals from the acoustic sensor 20 and the output data comprises detection of any cuff fastening slippage event, and preferably also a timing, and duration of the event.

Suitable machine-learning algorithms for being employed in the present invention will be apparent to the skilled person. Examples of suitable machine-learning algorithms include decision tree algorithms and artificial neural networks. Other machine-learning algorithms such as logistic regression, support vector machines or Naive Bayesian models are suitable alternatives.

The structure of an artificial neural network (or, simply, neural network) is inspired by the human brain. Neural networks are comprised of layers, each layer comprising a plurality of neurons. Each neuron comprises a mathematical operation. In particular, each neuron may comprise a different weighted combination of a single type of transformation (e.g. the same type of transformation, sigmoid etc. but with different weightings). In the process of processing input data, the mathematical operation of each neuron is performed on the input data to produce a numerical output, and the outputs of each layer in the neural network are fed into the next layer sequentially. The final layer provides the output.

Methods of training a machine-learning algorithm are well known. Typically, such methods comprise obtaining a training dataset, comprising training input data entries and corresponding training output data entries. An initialized machine-learning algorithm is applied to each input data entry to generate predicted output data entries. An error between the predicted output data entries and corresponding training output data entries is used to modify the machine-learning algorithm. This process can be repeated until the error converges, and the predicted output data entries are sufficiently similar (e.g. ±1%) to the training output data entries. This is commonly known as a supervised learning technique.

For example, where the machine-learning algorithm is formed from a neural network, (weightings of) the mathematical operation of each neuron may be modified until the error converges. Known methods of modifying a neural network include gradient descent, backpropagation algorithms and so on.

The training input data entries correspond to example sample sound signals or sound recordings. The training output data entries correspond to an indication as to whether each sound recording contains an instance of a slippage event, and preferably also an indication of a timing and duration of the event within the signal.

There are various different options for the response action which is triggered by the control unit 12 responsive to the detection of a cuff fastening slippage event.

In accordance with one or more embodiments, the response action may comprise processing the pressure signal acquired from the cuff 14 to identify one or more artifacts in the pressure signal corresponding to the detected cuff fastening slippage events. When slippage of the cuff fastening occurs, this results in a sudden drop in the pressure applied to the measurement site by the cuff, due to the sudden loosening of the cuff wrapping. This sudden drop in applied pressure then manifests in an oscillation in the cuff pressure signal. Such oscillations can be easily confused with the pulsatile oscillations associated with arterial blood pressure pulses. Thus, when cuff slipping occurs, these slippage oscillations can lead to false readings for blood pressure measurements.

Thus, in an advantageous set of embodiments, the control unit 12 is configured to detect artefacts in the signal in the form of oscillations in the signal associated with the slippage events.

The identifying of these artifacts may be based on a process of correlating timings of the detected slippage events (in the acoustic signal) to the acquired pressure signal to detect signal elements (e.g. signal oscillations) which temporally correspond with the slippage events. Thus, the detected timings of the slippage events in the acoustic signal can be used to temporally locate artifacts in the sound signal associated with the slippage events.

Once these have been identified, the response action may further comprise processing the acquired pressure signal to compensate for the detected artifacts.

For example, the compensating may comprise discarding the identified oscillations in the acquired pressure which temporally correspond with the occurrence of the slippage event. Thus, in effect the control unit 12 is configured to discard the acquired pressure signal at occasions where it is distorted by the detected artefact. In this way, the artefact is prevented from affecting the determined blood pressure measurement.

Additionally or alternatively, in some embodiments the control unit may be configured to compensate for the detected artefact by applying a correction to the acquired pressure signal at the one or more occasions where the detected artefact occurs (e.g. to suppress or remove or eliminate the detected artefact in the acquired pressure signal). Thus, in effect, the control unit 12 can be configured to correct the effect of cuff slippage on the acquired pressure signal. In this way, the artifact is prevented from affecting the determined blood pressure measurement signal.

The correction may be determined in the pressure domain (e.g. from the raw data of the acquired pressure signal) or in the pressure rate domain (e.g. from the derivative of the acquired pressure signal). In the pressure domain, the correction may be determined as an inverse of the artifact detected in the raw data of the acquired pressure signal. In the pressure rate domain, the correction may be determined as an inverse of the artifact detected in the derivative of the acquired pressure signal.

In some embodiments, where the correction is determined in the pressure domain, the correction may be applied directly to the raw data of the acquired pressure signal. In some embodiments where the correction is determined in the pressure rate domain, the correction may either be applied to (e.g. subtracted from) the derivative of the acquired pressure signal directly, or integrated to acquire a correction in the pressure domain and then be applied to (e.g. subtracted from) the raw data of the acquired pressure signal.

In some embodiments, where the correction is applied to the raw data of the acquired pressure signal, the control unit 12 can be configured to apply the correction to the acquired pressure signal by subtracting the detected artifact from the raw data of the acquired pressure signal at one or more occasions (e.g. events) where the detected artifact occurs to acquire a corrected pressure signal. The subtraction of the detected artifact from the raw data of the acquired pressure signal can, for example, comprise adding an inverse of the detected artifact to the raw data of the acquired pressure signal.

In embodiments, where the correction is applied to the derivative of the acquired pressure signal, the control unit 12 can be configured to apply the correction to the acquired pressure signal by subtracting the detected artifact from the derivative of the acquired pressure signal (i.e. from the pressure rate signal) at one or more occasions where the detected artifact occurs and integrating the derivative of the acquired pressure signal with the detected artifact subtracted to acquire a corrected pressure signal. The subtraction of the detected artifact from the derivative of the acquired pressure signal can, for example, comprise adding an inverse of the detected artifact to the derivative of the acquired pressure signal.

In some embodiments, the apparatus 12 can be configured to perform the process of detecting the slippage events and performing the one or more response actions in real-time with acquisition of the pressure signal from the cuff during inflation of the cuff.

In further embodiments, the response action may additionally or alternatively take one or more different forms.

For example, in accordance with one or more embodiments, the response action may comprise adjusting a duration or repetition count of the blood pressure measurement performed using the cuff. For example, in the event that a cuff slippage event is detected, the blood pressure measurement may be extended in duration, i.e. not stopped too early, so that, in effect, additional blood pressure measurement data can be acquired to compensate for the presence of earlier data compromised by the slippage event. Additionally or alternatively, the blood pressure measurement may be deliberately repeated one or more times in full in the event of detection of one or more slippage events, to avoid the slippage events compromising the whole blood pressure measurement.

Additionally or alternatively, the response action may comprise chording occurrences slippage events and labelling acquired pressure measurements which overlap with the detected slippage events accordingly. This thereby alerts a person with viewing the blood pressure measurements to the fact that the measurement data may be compromised or accurate due to the occurrence of the slippage event.

Examples in accordance with a further aspect of the invention provide a blood pressure measurement system which comprise a wearable blood pressure measurement cuff 14 (such as in accordance with the descriptions above); and an apparatus 10 in accordance with any example or embodiment outlined above or described below, or in accordance with any claim of this application. The control unit 12 of the apparatus 10 is arranged operatively coupled with the blood pressure measurement cuff for acquiring from the cuff said pressure signal.

Examples in accordance with a further aspect of the invention provide a method for use in determining a blood pressure and/or pulse rate measurement using a wearable cuff, wherein the wearable cuff is inflatable for pressurizing a measurement site of the subject.

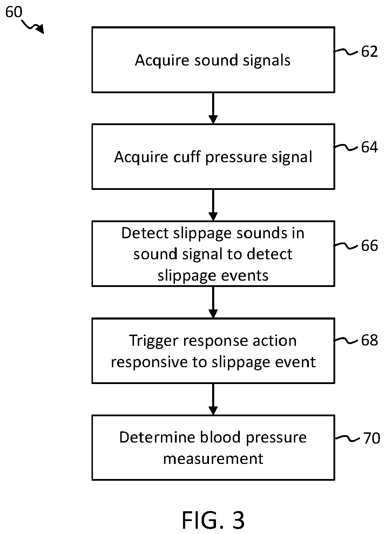

An example method in accordance with one or more embodiments is outlined in block diagram form in Fig. 3.

The method 60 comprises:
acquiring 62 sound signals corresponding to sounds originating from the blood pressure measurement cuff;
acquiring 64 from the wearable cuff a pressure signal indicative of a pressure in the wearable cuff during inflation of the cuff,
analyzing 66 the acquired sound signals to detect sounds corresponding to slippage of a fastening arrangement of the cuff, and thereby identify occurrence of cuff fastening slippage events;
triggering 68 a response action responsive to detecting any cuff fastening slippage events; and
determining 70 a blood pressure measurement of the subject using an oscillometric technique, by analyzing oscillations in the acquired pressure signal.

Implementation options and details for each of the above steps may be understood and interpreted in accordance with the explanations and descriptions provided above for the apparatus aspect of the present invention (i.e. the apparatus aspect).

Any of the examples, options or embodiment features or details described above in respect of the apparatus aspect of this invention (in respect of the apparatus 10) may be applied or combined or incorporated into the present method aspect of the invention.

Examples in accordance with a further aspect of the invention provide a computer program product comprising code means configured, when run on a processor, to cause the processor to perform a method in accordance with in accordance with any example or embodiment outlined above or described below, or in accordance with any claim of this application.

The processor is a processor operatively coupled with a wearable blood pressure measurement cuff, and further operatively coupled with an acoustic sensor for acquiring sounds originating from the wearable cuff.

As discussed above, embodiments make use of one or more processors to perform various functions. For example, the control unit 12 may comprise a processor unit 12. The processor can be implemented in numerous ways, with software and/or hardware, to perform the various functions required. The processor typically employs one or more microprocessors that may be programmed using software (e.g., microcode) to perform the required functions. The processor may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs).

In various implementations, the processor may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

A single processor or other unit may fulfill the functions of several items recited in the claims.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to".

Any reference signs in the claims should not be construed as limiting the scope.

## Claims

1. An apparatus (10) for use with a wearable cuff (14) in determining blood pressure, wherein the wearable cuff is inflatable for pressurizing a measurement site of the subject, the apparatus comprising:
an acoustic sensor (20) for detecting sounds corresponding to slippage of a fastening arrangement of the cuff; and
a control unit (12) operatively coupleable with the cuff and configured to
acquire a pressure signal indicative of a pressure in the wearable cuff during inflation of the cuff,
receive sound signals from the acoustic sensor,
analyze the sound signals to detect sounds corresponding to slippage of a fastening arrangement of the cuff; and thereby identify occurrence of cuff fastening slippage events, and
trigger a response action responsive to detecting any cuff fastening slippage events,
wherein the control unit is further operable to determine the blood pressure of the subject using an oscillometric technique, by analyzing oscillations in the acquired pressure signal.

2. The apparatus (10) as claimed in claim 1, wherein the apparatus is for use with a wearable cuff (14) comprising a hook and loop type fastening arrangement (15), and wherein the control unit is configured to detect in the sound signals sounds corresponding to slippage of a hook-and-loop type fastening.

3. The apparatus (10) as claimed in claim 1 or 2, wherein the acoustic sensor (20) is comprised in the control unit (12).

4. The apparatus (10) as claimed in claim 3, wherein the acoustic sensor (20) is arranged in acoustic communication with a connection port (32) for connecting the control unit (12) to the cuff (14) in use, and for example wherein the acoustic sensor is arranged in direct or indirect physical communication with connection port.

5. The apparatus (10) as claimed in claim 4, wherein the acoustic sensor (20) is comprised by the control unit (12) and arranged in acoustic communication with a fluid supply connection (32) for fluidly connecting to the wearable cuff (14) in use for supplying fluid for controlling inflation of the cuff.

6. The apparatus (10) as claimed in any of claims 1-5, wherein the analyzing the sound signals to detect the slippage events comprises detecting in the acquired sound signals one or more pre-determined signal features or characteristics associated with cuff slippage, or one or more pre-determined cuff-slippage signatures or fingerprints in the signals.

7. The apparatus (10) as claimed in any of claims 1-6, wherein the analyzing the sound signals to detect the slippage events is based on use of a machine learning algorithm.

8. The apparatus (10) as claimed in any of claims 1-7, wherein the response action comprises processing the acquired pressure signal to identify one or more artifacts in the pressure signal corresponding to the detected cuff fastening slippage events.

9. The apparatus (10) as claimed in claim 8, wherein identifying the artifacts is based on correlating timings of the detected slippage events to the acquired pressure signal to detect signal elements which temporally correspond with the slippage events.

10. The apparatus (10) as claimed in claim 8 or 9, wherein the response action comprises processing the acquired pressure signal to compensate for the detected artifacts.

11. The apparatus (10) as claimed any of claims 1-10, wherein the response action comprises adjusting a duration or repetition count of the blood pressure measurement performed using the cuff (14).

12. The apparatus (10) as claimed in any of claims 1-11, wherein the control unit (12) comprises a fluid supply means (36), and a fluid supply outlet port (32) for fluidly connecting to the wearable cuff (14) in use, and the control unit is configured to implement an oscillometric blood pressure measurement using the wearable cuff, based on controlling the inflation of the cuff, and acquiring the pressure signal indicative of the pressure inside the cuff.

13. A system, comprising
a wearable blood pressure measurement cuff (14),
an apparatus (10) as claimed in any of claims 1-12, the control unit of the apparatus arranged operatively coupled with the blood pressure measurement cuff for acquiring from the cuff said pressure signal.

14. A method (60) for use in determining a blood pressure and/or pulse rate measurement using a wearable cuff, wherein the wearable cuff is inflatable for pressurizing a measurement site of the subject, the method comprising:
acquiring (62) sound signals corresponding to sounds originating from the blood pressure measurement cuff;
acquiring (64) from the wearable cuff a pressure signal indicative of a pressure in the wearable cuff during inflation of the cuff;
analyzing (66) the acquired sound signals to detect sounds corresponding to slippage of a fastening arrangement of the cuff, and thereby identify occurrence of cuff fastening slippage events;
triggering (68) a response action responsive to detecting any cuff fastening slippage events; and
determining (70) a blood pressure measurement of the subject using an oscillometric technique, by analyzing oscillations in the acquired pressure signal.

15. A computer program product comprising code means configured, when run on a processor, to cause the processor to perform a method according to claim 14,
wherein said processor is a processor operatively coupleable with a wearable blood pressure measurement cuff (14), and further operatively coupleable with an acoustic sensor (20) for acquiring sounds originating from the wearable cuff.
